Europäisches Patentamt

European Patent Office

Office européen des brevets

(11) Veröffentlichungsnummer: **0 297 445 B1**

(12) **EUROPÄISCHE PATENTSCHRIFT**

(45) Veröffentlichungstag der Patentschrift: **12.02.92**

(51) Int. Cl.5: **C07C 57/05**, C07C 57/055

(21) Anmeldenummer: **88110065.5**

(22) Anmeldetag: **24.06.88**

(54) Verfahren zur Herstellung von Methacrylsäure.

(30) Priorität: **02.07.87 DE 3721865**

(43) Veröffentlichungstag der Anmeldung:
**04.01.89 Patentblatt 89/01**

(45) Bekanntmachung des Hinweises auf die
Patenterteilung:
**12.02.92 Patentblatt 92/07**

(84) Benannte Vertragsstaaten:
**BE DE ES FR GB IT NL**

(56) Entgegenhaltungen:
**DE-A- 3 002 660**
**DE-C- 875 194**
**GB-A- 2 024 201**

(73) Patentinhaber: **BASF Aktiengesellschaft**
**Carl-Bosch-Strasse 38**
**W-6700 Ludwigshafen(DE)**

(72) Erfinder: **Krabetz, Richard, Dr**
**Unterer Waldweg 8**
**W-6719 Kirchheim(DE)**
Erfinder: **Duembgen, Gerd, Dr.**
**Auerstrasse 14**
**W-6700 Ludwigshafen(DE)**
Erfinder: **Merger, Franz, Dr.**
**Max-Slevogt-Strasse 25**
**W-6710 Frankenthal(DE)**
Erfinder: **Jaeger, Michael**
**Holzgasse 22**
**W-6707 Schifferstadt(DE)**
Erfinder: **Thiessen, Fritz, Dr.**
**Friedlandweg 15**
**W-6700 Ludwigshafen(DE)**
Erfinder: **Vogel, Herbert, Dr.**
**Wolfsgrubenweg 17**
**W-6700 Ludwigshafen(DE)**

EP 0 297 445 B1

**Beschreibung**

Es ist bekannt, Methacrylsäure durch Gasphasenoxidation von Methacrolein oder Isobutyraldehyd mit Sauerstoff- und Wasserdampfethaltenden Gasgemischen an Molybdän- und Phosphor-haltigen Katalysatoren herzustellen.

Die meisten bekannten Verfahren zur Herstellung von Methacrylsäure gehen von Methacrolein aus, das durch katalytische Oxidation von Isobutylen oder tert. Butanol erhalten wird. In dem als "first-second connected process" bezeichnetem Verfahren wird das Reaktionsgas der primären Oxidationsstufe, in der Isobutylen oder tert. Butanol zu Methacrolein umgesetzt wurde, ohne Isolierung des gebildeten Methacroleins direkt in die sekundäre Oxidationsstufe eingespeist, in der das Methacrolein zu Methacrylsäure an Mo- und P-haltigen Heteropolysäuren oxidiert wird. Bei dem Alternative "first-second separated process" wird das in der primären Oxidationsstufe erzeugte Methacrolein aus dem Reaktionsgas abgetrennt, mit $O_2$ bzw. Luft, Wasserdampf und einem Inertgas gemischt und dann der sekundären Oxidationsstufe zugeführt. Andere Verfahren gehen von Isobutyraldehyd oder von Methacrolein aus, das durch Kondensation von Propanal und Formaldehyd in Gegenwart von sek. Aminen oder Aminalen als Katalysatoren erhalten wurde. Die Problematik bei allen genannten Prozessen besteht darin, daß

a) mit dem in den primären Stufen erzeugten Methacrolein auch reaktionsbedingt hochsiedende Nebenprodukte gebildet werden, die die Aktivität und Selektivität der Katalysatoren der sekundären Oxidationsstufe reduzieren und durch Abscheidung in feindisperser Form einen Anstieg des Druckverlustes verursachen.

b) mit den bekannten Katalysatoren für die Methacrolein-Oxidation kein vollständiger Umsatz des Methacroleins in der sekundären Oxidationsstufe erreicht wird, so daß das nicht umgesetzte Methacrolein aus wirtschaftlichen Gründen isoliert und in die Oxidationsstufe zurückgeführt werden muß. Die starke Neigung des in der sekundären Oxidationsstufe erzeugten, Methacrolein und ungesättigte Hochsieder enthaltenden Produktgemisches zu polymerisieren und schwer abtrennbare Aerosole zu bilden, führt leicht zu Feststoffabscheidungen in den nachfolgenden Kühlzonen und Wärmetauschern und damit zu einem Anstieg der Druckverluste nach oft kurzen Betriebszeiten. Außerdem können die Hochsieder als Aerosole mit den rückgeführten Methacrolein-haltigen Gasströmen in die Oxidationsstufe gelangen und dort die unter (a) angeführten Schwierigkeiten auslösen.

Es wurden verschieden Vorschläge zur Lösung dieser Probleme gemacht, die jedoch nicht befriedigen und im allgemeinen nur auf die 2-Stufen-Prozesse auf Basis Isobutylen-tert. Butanol ausgerichtet sind.

Für den "first-second connected process" wird beispielsweise in der US-PS 4 558 028 vorgeschlagen, druckverlustarme Hohlzylinder einer definierten Zusammensetzung als Katalysatoren in der Methacrolein-Oxidationsstufe einzusetzen, um die negativen Auswirkungen einer Abscheidung von hochsiedenden Nebenprodukten, wie Terephthalsäure und teerartiger Gemische, zu mildern. Durch diese Maßnahme wird jedoch keine grundsätzliche Lösung des Gesamtproblems erreicht, da die Adsorption bzw. Abscheidung auf dem Katalysator nicht verhindert wird. Um die Polymerisatabscheidung in den kritischen Kühlzonen nach der sekundären Oxidationsstufe und das Durchschlagen von Aerosolen zu unterbinden, wird in der GB-PS 2 116 963 für den gleichen Prozeß vorgeschlagen, das auf 250°C abgekühlte Reaktionsgas aus dem Oxidationsreaktor in einem mit Siebböden ohne Ablaufstutzen versehenen Kühlturm mit einer wäßrigen Methacrylsäurelösung rasch auf etwa 50°C abzukühlen und das als Aerosol die Kühlzone verlassende Abgas in einem Venturiwäscher bei etwa 40°C mit einer wäßrigen Methacrylsäurelösung zu waschen, bevor es einem Methacrylsäure-Absorber zugeführt wird. Ein Teil des Methacrolein-haltigen Abgases des Absorbers wird in einem dritten Oxidationsreaktor umgesetzt, aus dem Restteil des Abgases wird in einem mit Wasser bei 10 bis 15°C betriebenen Absorber das Methacrolein ausgewaschen. Insgesamt ist das Verfahren der GB-PS 2 116 963 apparativ sehr aufwendig und mit Wertproduktverlusten von mindestens 1 Mol% verbunden.

In den für den "first-second separated process" typischen Verfahren der GB-PS 2 041 930 und 2 045 759 wird das in der primären Oxidationsstufe aus Isobutylen erzeugte Methacrolein zusammen mit der Hauptmenge der organischen Nebenprodukte in zwei Absorptionsstufen aus dem Reaktionsgas ausgewaschen, gemäß der GB-PS 2 041 930 als wäßrige Lösung auf das obere Ende einer Abstreifkolonne aufgegeben und dort mit Kreisgas der sekundären Oxidationsstufe bei Sumpftemperaturen von 120°C ausgetrieben. Das mit Methacrolein und Wasserdampf beladene Kreisgas wird direkt dem Oxidationsreaktor zugeführt. Auch bei diesem Verfahren kann nicht ausgeschlossen werden, daß als Katalysatorgift wirkende hochsiedende Nebenprodukt in die sekundäre Oxidationsstufe gelangen, da die wäßrige Methacroleinlösung am oberen Ende der Abstreifkolonne zugegben wird. Das Reaktionsgas des sekundären Oxidationsreaktors wird gemäß der GB-PS 2 045 759 direkt oder nach indirekter Abkühlung auf 150°C in einen Kühlturm eingeführt und dort mit einem im Kreis geführtem Flüssigkeitsstrom auf etwa 40°C abgeschreckt, das

2

Methacrolein-, Wasser-und Nebenprodukte enthaltenden Abgas des Kühlturms wird in die Oxidationsstufe zurückgeführt. Nach den Angaben der GB-PS 2 116 963 sind die Maßnahmen der GB-PS 2 045 759 nicht geeignet, die Abscheidung von polymeren Feststoffen in den Kühlzonen und die Bildung von Aerosolen bzw. deren Einführung in den Oxidationsreaktor zu verhindern.

Die EP-OS 194 620 beschreibt ein Verfahren zur Herstellung von Methacrylsäure durch Gasphasenoxidation von Methacrolein, das durch Umsetzen von Propanal mit Formaldehyd gewonnen ist und das als störende bzw. hochsiedende Nebenprodukte u.a. 2-Methylpentenal, dimeres Methacrolein, 2-Methylpentanal, 3-Methoxy-isobutyraldehyd neben 0,5 bis 3 Gew.% Wasser und geringe Mengen an Methanol, Formaldehyd und Propionaldehyd enthält. Es wird empfohlen, das Methacrolein vor der Einführung in den Oxidationsreaktor durch Absorption oder Destillation so zu reinigen, daß die Konzentration der sauerstoffhaltigen und/oder ungesättigten Verbindungen mit mehr als 4 C-Atomen in der Gasmischung kleiner als 0,2 Gew.%, bezogen auf das Methacrolein, ist. Im übrigen sind dieser EP-OS keine Maßnahmen zur Verhinderung von Feststoffabscheidungen in den kritischen Kühlzonen und der Aerosolbildung bei der Isolierung der Methacrylsäure und des nicht umgesetzten Methacroleins zu entnehmen, die über die bereits besprochenen Vorschläge hinausgehen.

Im Laufe der weiteren Bearbeitung des zuletzt genannten Verfahrens hat es sich gezeigt, daß eine Vorreinigung des Methacroleins gemäß der Lehre der EP-OS 194 620 nicht völlig ausreicht, um im technischen Dauerbetrieb temporäre oder irreversible Vergiftungen der Oxidationskatalysatoren durch Verunreinigungen des Methacroleins auszuschließen, wenn die bekannten Maßnahmen zur Isolierung der Methacrylsäure und des nicht umgesetzten Methacroleins sowie der Einführung des frischen Methacroleins in die Oxidationsstufe angewandt werden.

Es wurde nun ein Verfahren zur Herstellung von Methacrylsäure durch Gasphasen-Oxidation von Methacrolein oder Isobutyraldehyd mit Sauerstoff-und Wasserdampf-enthaltenden Gasgemischen an Molybdän- und Phosphorenthaltenden Katalysatoren bei Temperaturen von 250 bis 400˚C gefunden, bei dem das heiße Reaktionsgas des Oxidationsreaktors auf Temperaturen unter 100˚C abgekühlt, zur Absorption der gebildeten Methacrylsäure durch eine mit Wasser bei Temperaturen unter 100˚C betriebene Absorptionskolonne (1) geleitet und nach Zumischen der den verbrauchten Mengen Methacrolein oder Isobutyraldehyd und Sauerstoff entsprechenden Mengen dieser Stoffe zum Teil in den Oxidationsreaktor zurückgeführt wird. Das Verfahren ist dadurch gekennzeichnet, daß eine Menge an Methacrolein oder Isobutyraldehyd die gleich der in der Reaktion verbrauchten Menge an Methacrolein oder Isobutyraldehyd ist, zusammen mit Polymerisationsinhibitoren dem Reaktorgas in den unteren zwei Dritteln der Absorptionskolonne (1) in flüssiger Form geführt und aus dem Reaktorabgas in Strömungsrichtung vor der Zuführungsstelle des frischen Methacroleins bzw. Isobutyraldehyds oder nach der Absorptionskolonne (1) ein Seitenstrom abgezweigt wird, aus dem in einer mit Wasser bei Temperaturen von 0 bis < 10˚C betriebenen Waschkolonne (2) das nicht umgesetzte Methacrolein oder Isobutyraldehyd ausgewaschen und als wäßrige Lösung auf den Kopf der Absorptionskolonne (1) aufgegeben wird. In einer bevorzugten Ausführungsform des Verfahrens wird das heiße Abgas des Oxidationsreaktors zunächst indirekt auf Temperaturen von 220 bis 260˚C gekühlt und dann mit der anfallenden wäßrigen Methacrylsäurelösung in einem mit Sprühdüsen versehenen Quenchrohr rasch auf Temperaturen von mehr als 70 bis etwa 80˚C abgekühlt. Als Polymerisationsinhibitoren sind z.B. Hydrochinon, Hydrochinonmonomethylether, Benzhochinon und ihre Kombinationen mit z.B. Diphenylamin geeignet.

Durch die erfindungsgemäßen Maßnahmen wird sowohl die Polymerisatabscheidung und die Bildung Methacrylsäure und Hochsieder enthaltender Aerosole in den Kühlzonen praktisch völlig unterdrückt, als auch eine temporäre oder irreversible Leistungsminderung der Oxidationskatalysatoren durch hochsiedende oder als Katalysatorgift wirkende leichtsiedende Verunreinigungen des frisch zugeführten Methacroleins bzw. Isobutyraldehyds weitgehend vermieden.

Das neue Verfahren zur Herstellung von Methacrylsäure durch Gasphasenoxidation von Methacrolein oder Isobutyraldehyd zeichnet sich durch geringe Verluste bei der Abtrennung der Methacrylsäure und der Rückgewinnung und Rückführung des nicht umgesetzten Methacroleins und/oder Isobutyraldehyds. Es wird im folgenden anhand der Skizzen 1 und 2 erläutert:

Das aus dem Oxidationsreaktor (4) austretende im allgemeinen 300 bis 340˚C heiße Reaktionsgas (7) wird in einer bevorzugten Ausführungsform zunächst in einem Wärmetauscher (5) auf 220 bis 260˚C, vorzugsweise auf 230 bis 250˚C gekühlt, wobei herkömmliche mit Druckwasser oder anderen Kühlmedien betriebene Wärmetauscher, beispielsweise Röhren-Wärmetauscher eingesetzt werden können, deren spezifische Wärmetausch-Fläche vorteilhaft mehr als 200 $m^2/m^3$ beträgt und die sich unmittelbar, d.h. ohne größeren nicht gekühlten Zwischenraum, an den unteren Rohrboden des Oxidationsreaktors anschließen, um eine nichtkatalytische Nachverbrennung des methacroleinhaltigen Reaktionsgases zu verhindern. Rohrbündelreaktoren für die katalytische Umsetzung des Methacroleins, deren am Gasausgang liegender und mit inerten Füllkörpern gefüllter Rohrteil einen getrennten Kühlmittelkreislauf aufweist, sind ebenfalls geeignet. Das vorgekühlte Reaktionsgas (8) wird anschließend in einem mit Sprühdüsen ausgerüsteten Quenchrohr (6) mit der anfallenden wäßrigen Methacrylsäurelösung rasch auf eine Temperatur von mehr als 70 bis etwa 80°C abgekühlt, bevor es in die Absorptionskolonne (1) für die Methacrylsäure eintritt. Es wurde überraschend gefunden, daß bei Einhaltung der Grenztemperatur von mehr als 70 bis 80°C im Rahmen des erfindungsgemäßen Verfahrens sowohl die Abscheidung von Polymerisat als auch die Bildung von Aerosolen praktisch vollständig unterdrückt wird. Im Gegensatz zu den Lehren der GB-PS 2 116 963 ist es dabei nicht erforderlich, den Quenchvorgang in einem mit Siebböden ohne Ablaufstutzen ausgerüsteten Kühlturm mit nachgeschaltetem Venturiwäscher vorzunehmen. Es kann zwar zweckmäßig sein, als Quenchrohr ein Venturirohr einzusetzen, um den Konakt zwischen der Gasphase und der Polymerisationsinhibitoren enthaltenden Flüssigphase zu intensivieren; diese Maßnahme ist jedoch nicht erfindungswesentlich, da sie die Aerosolbildung nicht verhindert, wenn die Quenchtemperatur merklich weniger als 70°C beträgt. Aus gem gequenchten Reaktionsgas (9) wird in dem Absorber (1) die Methacrylsäure zusammen mit mittel- und hochsiedenden Nebenprodukten, wie Essig-, Propion-, Acryl-, Malein-, Fumar- und Citraconsäure, Formaldehyd und Ameisensäure bei Temperaturen von mehr als 70 bis etwa 80°C und Drücken von etwa 1,1 bis 2 bar ausgewaschen. Die Art des Absorbers (1) ist nicht erfindungswesentlich. Geeignet sind z.B. mit Füllkörpern, wie Raschigringen, gefüllte Kolonnen sowie auch Sieb- oder Lochbodenkolonnen mit oder ohne Ablaufstutzen. Als Absorptionsmittel dient Wasser, das zusammen mit Polymerisationsinhibitoren zunächst auf den Kopf des Absorbers (2) aufgegeben wird, um das nicht umgesetzt Methacrolein aus dem Zweigstrom (10) bzw. (11) und (12) des Reaktionsgases auszuwaschen; das methacroleinhaltige Abwasser (13) des Absorbers (2) wird auf den Kopf bzw. die oberen Böden des Methacrylsäure-Absorbers (1) gepumpt, wo das gelöste Methacrolein desorbiert und zusammen mit dem wasserdampfgesättigten Abgas des Absorbers (1) in den Oxidationsreaktor (4) als Kreisgas (14) zurückgeführt wird. Ein erfindungswesentliches Merkmal ist die Einleitung des frischen Methacroleins in die Absorptionskolonne (1). Es wurde überraschend gefunden, daß die Leistung der bekannten Katalysatoren für die Methacroleinoxidation nicht unabhängig davon ist, in welcher Form und an welcher Stelle das Methacrolein in den Oxidationsreaktor eingeführt wird. Das gilt inbesondere für ein Methacrolein, das durch Kondensation von Propanal mit Formalehyd in Gegenwart von sek. Aminen oder Aminalen hergestellt wird. Wurde das Methacrolein nach dem Absorber (1) für die Methacrylsäure durch Verdampfung in das Kreisgas eingebracht, so war die Katalysatorleistung schlechter, als wenn das Methacrolein flüssig oder als wäßrige Lösung, d.h. in flüssiger Form, in die unteren zwei Drittel der Absorptionskolonne (1) eingeführt wurde.

Die Abzweigung eines Seitenstroms (10) bzw. (11) aus dem Reaktionsgas ist notwendig, um die gebildeten Kohlenoxide mit dem nicht verbrauchten Sauerstoff und den nicht kondensierbaren Inertgasen auszuschleusen. Sie können einer katalytischen oder thermischen Verbrennungsanlage zugeführt werden. Der Seitenstrom kann wahlweise nach dem Methacrylsäureabsorber (1) oder unterhalb der Zuführungsstelle für das frische Methacrolein abgezweigt werden. Im letzten Fall wird der Zweigstrom zunächst in den Absorber (3) geleitet, um die Methacrylsäure mit Wasser bei Temperaturen unter 70°C auszuwaschen, bevor er dem Absorber (2) zugeführt wird. Ein Vorteil dieser bevorzugten Ausführungsform ist z.B. die Senkung des Betriebsdruckes.

Die aus dem Sumpf der Absorber (1) und (3) abgezogene 10 bis 20 %ige wäßrige Methacrylsäurelösung (16) enthält im allgemeinen nur geringe Mengen Methacrolein von maximal 3 Gew.%, bezogen auf die in der Lösung enthaltene Methacrylsäure. Diese Restmenge an Methacrolein kann z.B. mit Luft oder Inertgasen ausgetrieben und dem Kreisgas zugemischt werden.

Das in den Oxidationsreaktor zurückgeführte Kreisgas enthält neben der erforderlichen Menge Methacrolein und Wasserdampf sehr geringe Mengen an Leichtsiedern, wie Actaldehyd, Aceton, Acrolein und Propionaldehyd. Nach Zumischen einer der verbrauchten Sauerstoffmenge gleichen Menge Sauerstoff als Reinsauerstoff oder Luft besitzt das Synthesegas meist die Zusammensetzung 3 bis 7 Vol% Methacrolein, 5 bis 10 Vol% $O_2$, 15 bis 30 Vol% $H_2O$, weniger als 0,1 Vol.% Leichtsieder und als Rest Inertgase, wie $N_2$, CO und $CO_2$.

Die Umsetzung des Methacroleins erfolgt im allgemeinen bei Temperaturen von 250 bis 400°C, Raumbelastungen von 500 bis 2000 $h^{-1}$ und Drücken von 1 bis 3 bar. Es hat sich als vorteilhaft erwiesen, die Raumbelastung und die Reaktionstemperatur so zu wählen, daß der Methacroleinumsatz im einfachen Durchgang 50 bis 80 Mol%, vorzugsweise 55 bis 75 Mol% beträgt. Die Katalysatoren enthalten im allgemeinen neben Mo und P noch andere Komponenten und weisen die Struktur von Heteropolysäuren und deren Salze auf. Besonders geeignete Katalysatoren enthalten Mo, P, V oder Nb, mindestens ein Alkali- und/oder Erdalkalimetall, ein oder mehrere Elemente aus der Gruppe Cu, Ag, Au, Fe, Co, Ni, Mn, Cr, Zr, Ge, Se, Te, Tl, In, Ga, Zn, Cd, Hg, seltene Erden, Rh, Re, Sb, U, B, As und/oder W und einen inerten Träger. Besonders wirksam unter den Betriebsbedingungen des erfindungsgemäßen Verfahrens haben sich Katalysatoren mit einer Zusammensetzung erwiesen, die durch die allgemeine Formel

$$Mo_{12} P_a V_b Nb_c Cs_d Cu_e M_{1f} M_{2g} M_{3h} M_{4i}$$

wiedergegeben wird
in der

| | | |
|---|---|---|
| $M_1$ | für K, Rb und/oder T1 | |
| $M_2$ | für Be, Mg, Ca, Sr und/oder Ba | |
| $M_3$ | für Fe, Ni, Co, Ag, Zn, Seltene Erden, Re, Mn, Ta, Ge, Si, Te, Se, In, W, Rh und/oder Sb | |
| $M_4$ | für As und/oder B | |
| a | für 0,1 bis 4 | |
| b | für 0 bis 4, vorzugsweise 0,05 bis 2 | |
| c | für 0 bis 4 | |
| b + c | für 0,05 bis 4 | |
| d | für 0 bis 3, vorzugsweise 0,5 bis 2 | |
| e | für 0 bis 2, vorzugsweise 0,05 bis 1 | |
| f | für 0 bis 3 | |
| g | für 0 bis 3 | |
| d + f | für 0,05 bis 3, vorzugsweise 0,5 bis 3 | |
| h | für 0 bis 2 | |
| e + h | für 0,05 bis 2, vorzugsweise 0,05 bis 1 | |
| i | für 0 bis 2 | |
| k | für die Anzahl der Sauerstoffatome, die für die formale Absättigung der Valenzen der übrigen Elemente erforderlich ist, stehen. | |

Derartige Katalysatoren sind verhältnismäßig unempfindlich gegen Verunreinigungen des Methacroleins und erzeugen weniger höhersiedende Aldehyde wie Furfurol, die bei der extraktiven und destillativen Reinigung der Methacrylsäure schlecht abzutrennen sind.

Die Vorteile des erfindungsgemäßen Verfahrens gegenüber den bekannten Verfahren werden durch das folgend Beispiel näher erläutert.

Beispiel

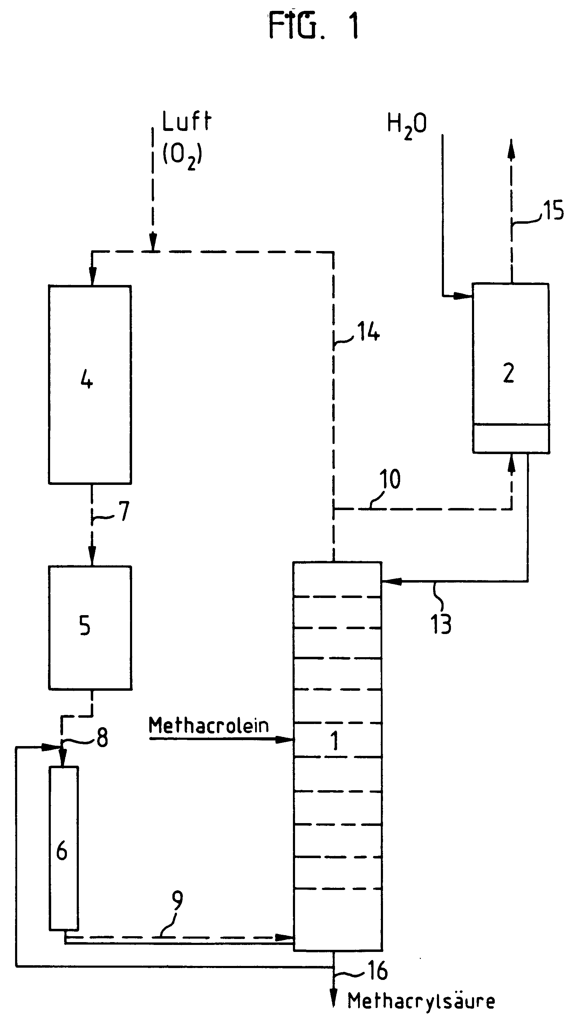

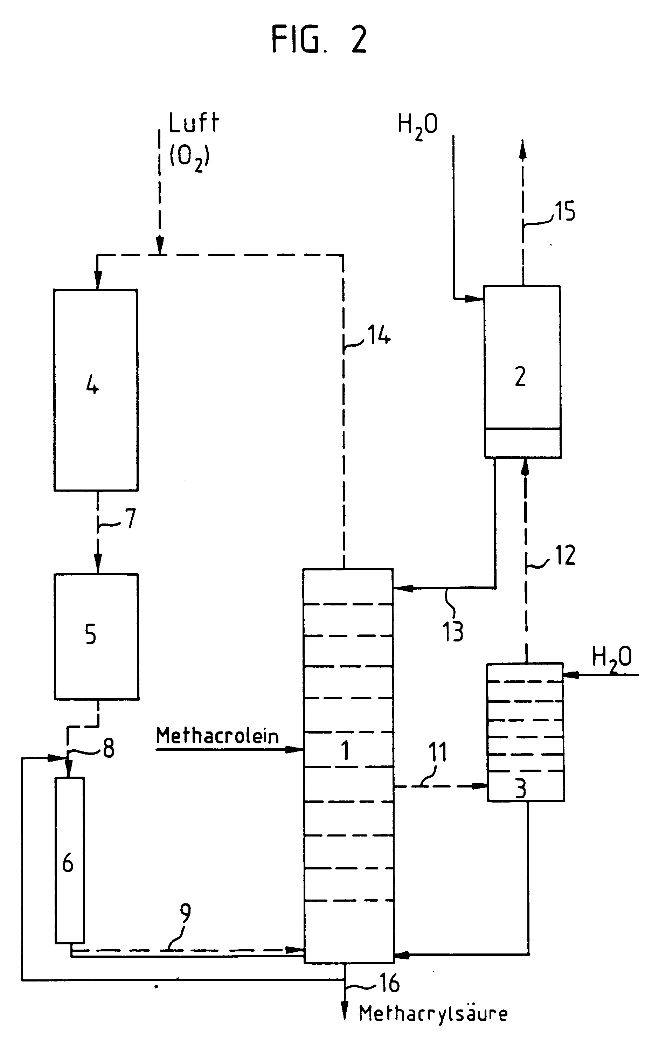

In einer Apparatur die der in Abb. 1 skizzierten apparativen Anordnung entspricht, wurde Methacrolein in der Gasphase an einem Katalysator der formelmäßigen Zusammensetzung

$$Mo_{12} P_{1,9} V_1 Cs_{1,9} Cu_{0,5} Rh_{0,008} O_x$$

zu Methacrylsäure oxidiert. Die Testbedingungen sind in der Tabelle 1 zusammengefaßt.

Tabelle 1

| Reaktor (4) | Rohrlänge 3 m, Durchmesser 26 mm |
|---|---|
| Druck Eingang Reaktor (4) | 1,35 bar |
| Reaktionstemperatur | 315° C |
| Katalysatorfüllmenge | 1 l (Hohlzylinder 6 x 6 x 2 mm) |
| Frischluftmenge | 258 Nl/Std. |
| Flüssig zugeführte Methacroleinmenge | 118 g/Std. |
| Kreisgasmenge | 900 Nl/Std. |
| Waschwasser auf Methacroleinabsorber (2) | 1104 g/Std. |
| Austrittstemperatur des Reaktionsgases aus dem Wärmetauscher (5) | 240° C |
| Gastemperatur am Ausgang Quenchrohr (6) | 80° C |
| Kopftemperatur des Methacrylsäure-Absorbers (1) | 72° C |
| Temperatur des Methacroleinabsorbers (2) | 4° C |

Das flüssig (unverdünnt) in der Mitte des Methacrylsäureabsorbers eingeführte Methacrolein enthielt 96,3 Gew.% Methacrolein, 2,1 Gew.% Wasser, 1,1 Gew.% Methanol, 0,2 Gew.% Propanal, 0,22 Gew.% Hochsieder, wie dimeres Methacrolein, sowie etwa 150 ppm N-haltige Verbindungen. Aus der Analyse des Abgasstromes aus dem Methacroleinabsorber (15) und der ausgeschleusten wäßrigen Methacrylsäurelösung (16) errechnete sich ein Umsatz von 98,5 Mol% und eine Methacrylsäureausbeute von 83,4 Mol%, beide Werte bezogen auf das frisch zugeführte Methacrolein. Der Umsatz im einfachen Durchgang betrug 59,9 Mol% und blieb über 4 Wochen Testzeit praktisch unverändert. Eine Aerosolbildung wurde nicht beobachtet. Der Druckverlust der Kolonnen und des Oxidationsreaktors blieb innerhalb der Testzeit konstant. Auch nach Austausch der Lochbodenkolonne gegen eine mit Raschigringen gepackte Füllkörperkolonne wurde kein Anzeichen für eine Feststoffablagerung in dem Methacrylsäureabsorber (1) festgestellt.

Vergleichsbeispiel a

Beispiel 1 wurde mit der Änderung wiederholt, daß das Methacrolein nicht flüssig in die Absorptionskolonne (1) eindosiert wurde, sondern über einen Verdampfer gasförmig in das Kreisgas eingebracht wurde. Innerhalb von 3 Tagen verschlechterte sich der Umsatz im einfachen Durchgang von 60 Mol% auf unter 50 Mol% unter stetigem Anstieg der Methacroleinkonzentration im Kreisgas (14) bzw. im Zweigstrom 10 und in der Sumpflösung des Methacroleinabsorbers (2).

Vergleichsbeispiel b

In einem weiteren Versuch wurde die Quenchtemperatur von 80° C auf 45° C abgesenkt. Diese Maßnahme bewirkte eine visuell erkennbare Nebelbildung in dem Abgasstrom des Quenchrohres. Das austretende Aerosol durchzog den Methacrylsäureabsorber und wurde mit dem Kreisgas zum Reaktor zurückgeführt. Der Druckverlust eines dem Reaktor vorgeschalteten Filters stieg innerhalb von 10 Tagen um 0,5 bar an. Der Versuch wurde daraufhin abgebrochen.

Vergleichsbeispiel c

In einem weiteren Versuch wurde die Kühlmitteltemperatur des Wärmetauschers (5) soweit abgesenkt, daß das Reaktionsgas auf eine Temperatur von 200° C abgekühlt wurde. Innerhalb einer Woche stieg daraufhin der Druckverlust des Wärmetauschers so stark an, daß der Versuch abgebrochen werden mußte. Bei der anschließenden Inspektion des Wärmetauscherrohres zeigte es sich, daß die Innenwand mit einer schwarzbraunen Feststoffmasse verkrustet war.

**Patentansprüche**

6

1. Verfahren zur Herstellung von Methacrylsäure durch Gasphasenoxidation von Methacrolein oder Isobutyraldehyd mit Sauerstoff- und Wasserdampfenthaltenden Gasgemischen an Molybdän- und Phosphorenthaltenden Katalysatoren bei Temperaturen von 250 bis 400° C, wobei das heiße Reaktionsgas des Oxidationsreaktors auf Temperaturen unter 100° C abgekühlt, zur Absorption der gebildeten Methacrylsäure durch eine mit Wasser bei Temperaturen unter 100° C betriebene Absorptionskolonne (1) geleitet und nach Zumischen der den verbrauchten Mengen Methacrolein oder Isobutyraldehyd und Sauerstoff entsprechenden Mengen dieser Stoffe zum Teil in den Oxidationsreaktor zurückgeführt wird, dadurch gekennzeichnet, daß eine Menge an Methacrolein oder Isobutyraldehyd, die gleich der Menge des in der Reaktion verbrauchten Methacrolein oder Isobutyraldehyd ist, zusammen mit Polymerisationsinhibitoren dem Reaktorabgas in den unteren zwei Dritteln der Absorptionskolonne (1) in flüssiger Form zugeführt und aus dem Reaktorabgas in Strömungsrichtung vor der Zuführungsstelle des frischen Methacroleins bzw. Isobutyraldehyd oder nach der Absorptionskolonne (1) ein Seitenstrom abgezweigt wird, aus dem in einer mit Wasser bei Temperaturen von 0 bis < 10° C betriebenen Waschkolonne (2) das nicht umgesetzte Methacrolein oder Isobutyraldehyd ausgewaschen und als wäßrige Lösung auf den Kopf der Absorptionskolonne (1) gegeben wird.

2. Verfahren nach Anspruch 1, dadurch gekennzeichnet, daß das heiße Reaktionsgas des Oxidationsreaktors zunächst indirekt auf Temperaturen von 220 bis 260° C abgekühlt und dann mit der anfallenden Methacrylsäurelösung in einem mit Sprühdüsen versehenen Quenchrohr rasch auf Temperaturen von mehr als 70 bis etwa 80° C abgekühlt wird.

3. Verfahren nach Anspruch 1 und 2, dadurch gekennzeichnet, daß der Seitenstrom des Reaktionsgases in Strömungsrichtung vor der Zuführungsstelle für das frische Methacrolein bzw. Isobutyraldehyd abgezweigt und zuerst zum Auswaschen der Methacrylsäure in eine mit Wasser bei weniger als 70° C betriebene Kolonne (3) geleitet und dann der Waschkolonne (2) zum Abtrennen von restlichem Methacrolein zugeführt wird.

4. Verfahren nach Anspruch 1 bis 3, dadurch gekennzeichnet, daß das Verfahren mit Methacrolein durchgeführt wird, das durch Kondensation von Propanal mit Formaldehyd in Gegenwart von sek. Aminen oder Aminalen hergestellt ist.

**Claims**

1. A process for preparing methacrylic acid by gas phase oxidation of methacrolein or isobutyraldehyde with an oxygen- and steam-containing gas mixture over a molybdenum- and phosphorus-containing catalyst at from 250 to 400° C where the hot reaction gas from the oxidation reactor is cooled down to below 100° C, is passed, for absorption of the methacrylic acid formed, through an absorption column (1) operated with water at below 100° C, and, after being replenished with quantities of methacrolein or isobutyraldehyde and oxygen corresponding to the quantities consumed of these substances, is partly recycled into the oxidation reactor, which comprises feeding a quantity of methacrolein or isobutyraldehyde equal to the quantity of methacrolein or isobutyraldehyde consumed in the reaction in liquid form together with a polymerization inhibitor into the reactor gas in the lower two-thirds of absorption column (1) and upstream of the feed point for the fresh methacrolein or isobutyraldehyde or downstream of absorption column (1) splitting off the reactor offgas a side stream which is washed in a wash column (2) operated with water at from 0 to < 10° C to remove unconverted methacrolein or isobutyraldehyde which is fed as an aqueous solution to the top of absorption column (1).

2. A process as claimed in claim 1, wherein hot reaction gas from the oxidation reactor is initially cooled down indirectly to from 220 to 260° C and then cooled down rapidly in a quench tube equipped with spray nozzles to from more than 70 to about 80° C with the methacrylic acid solution obtained.

3. A process as claimed in claim 1 or 2, wherein the side stream of the reaction gas is branched off upstream of the feed point for fresh methacrolein or isobutyraldehyde and first passed into a column (3) operated with water at below 70° C to wash out the methacrylic acid and then into a wash column (2) for separating off remaining methacrolein.

4. A process as claimed in claim 1 or 2 or 3, which is carried out with methacrolein prepared by condensation of propanal with formaldehyde in the presence of a secondary amine or aminal.

EP 0 297 445 B1

**Revendications**

1. Procédé de préparation de l'acide méthacrylique par oxydation en phase gazeuse de la méthacroléine ou de l'isobutyraldéhyde avec des mélanges de gaz contenant de l'oxygène et de la vapeur d'eau, sur des catalyseurs contenant du molybdène et du phosphore, à des températures de 250 à 400°C, conformément auquel on refroidit les gaz de réaction chauds du réacteur d'oxydation jusqu'à des températures inférieures à 100°C et on les fait passer à travers une colonne d'absorption (1) travaillant avec de l'eau à des températures inférieures à 100°C, en vue de l'absorption de l'acide méthacrylique formé, et on les renvoie en partie au réacteur d'oxydation après mélange aux proportions de méthacroléine ou d'isobutyraldéhyde et d'oxygène correspondant aux proportions consommées de ces substances, caractérisé en ce que l'on amène une proportion de méthacroléine ou d'isobutyraldéhyde, qui est égale à la proportion de la méthacroléine ou de l'isobutyraldéhyde consommé au cours de la réaction, en même temps que des inhibiteurs de polymérisation au gaz qui quitte le réacteur, sous forme liquide, dans les deux tiers inférieurs de la colonne d'absorption (1) et en ce qu'à partir du gaz qui quitte le réacteur, on prélève, dans la direction d'écoulement et avant l'endroit d'introduction de la méthacroléine fraîche ou de l'isobutyraldéhyde frais, ou après la colonne d'absorption (1) un courant latéral dont on lixivie la méthacroléine non convertie ou l'isobutyraldéhyde non converti dans une colonne de lavage (2) travaillant avec de l'eau à des températures de 0 à moins de 10°C et l'on introduit la méthacroléine ou l'isobutyraldéhyde non converti ainsi lixivié en tête de la colonne d'absorption (1) sous forme de solution aqueuse.

2. Procédé suivant la revendication 1, caractérisé en ce que l'on refroidit d'abord indirectement le gaz de réaction chaud du réacteur d'oxydation jusqu'à des températures de 220 à 260°C et on le refroidit ensuite rapidement à l'aide de la solution d'acide méthacrylique présente dans un tube de refroidissement brusque pourvu d'ajutages de pulvérisation, jusqu'à des températures de plus de 70°C et allant jusqu'à environ 80°C.

3. Procédé suivant les revendications 1 et 2, caractérisé en ce que l'on prélève le courant latéral du gaz de réaction dans la direction d'écoulement, avant l'endroit d'introduction de la méthacroléine fraîche ou de l'isobutyraldéhyde frais et on le conduit d'abord en vue de la lixiviation de l'acide méthacrylique dans une colonne (3) travaillant avec de l'eau à moins de 70°C et on l'amène ensuite dans la colonne de lavage (2) en vue de la séparation de la méthacroléine résiduelle.

4. Procédé suivant les revendications 1 à 3, caractérisé en ce qu'on le met en oeuvre avec de la méthacroléine préparée par condensation du propanal et du formaldéhyde en présence d'aminals ou d'amines secondaires.

8

# FIG. 1

Luft ($O_2$)

$H_2O$

Methacrolein

Methacrylsäure

# FIG. 2